# EUROPEAN PATENT APPLICATION

(11) **EP 1 887 089 A1**
(43) Date of publication of application: **13.02.2008**
(21) Application number: 06731049.0
(22) Date of filing: 04.04.2006
(51) Int. Cl.: C12Q 1/68, G01N 21/78, G01N 33/53, C12N 15/09

(54) **METHOD OF DETECTING HYBRIDIZATION BY USING INTERCALATOR**

(30) Priority: 20.04.2005 JP 2005121964
(71) Applicant: Sony Corporation, Minato-ku Tokyo 108-0075 (JP)
(72) Inventor: NEGISHI, Makiko c/o SONY CORPORATION, Tokyo 1410001 (JP)
(74) Representative: Jackson, Jonathan Andrew
(86) International application number: PCT/JP2006/307101
(87) International publication number: WO 2006/114995

(57) **Abstract**

To provide a novel and useful technique capable of achieving an improve S/N ratio in a hybridization detection technique using an intercalator. A method is provided wherein a hybridization is detected by measuring a fluorescence intensity from an intercalator I binding to a probe nucleic acid P and a complementary strand site of a target nucleic acid T. In the invention, a single-stranded target nucleic acid Ts, a single-stranded probe nucleic acid Ps forming no complementary strand and surplus nucleic acid moieties T₁₁, T₁₂ of a target nucleic acid T, which induce generation of noise fluorescence owing to the nonspecific binding of the intercalator I thereto, are digested and degraded with an enzyme having nuclease activity, such as a nucleolytic enzyme (nuclease) or the like.

## Description

### [Technical Field]

This invention relates to a technique of contributing to an improvement in accuracy of hybridization detection using an intercalator. More particularly, the invention relates to a technique of achieving an improvement in S/N ratio by degrading a single-stranded target nucleic acid or the like, which emits noise fluorescence caused by nonspecific binding of an intercalator, by enzyme digestion.

### [Technical Background]

In recent years, integrated circuits for bioassay called DNA chip or DNA microarray (hereinafter referred herein to generically as "DNA chip") wherein a given type of DNA is microarrayed according to a microarray technique are utilized for genetic variation analysis, analysis of SNPs (monobasic polymorphism), gene expression frequency analysis and the like and now begin to be widely put to practical use in the fields of drug discovery, clinical diagnosis, pharmacogenomics, studies on evolution, forensic pathology and the like.

The "DNA chip" is characterized in that because many types and a great number of DNA oligochains or probe nucleic acids such as of cDNA (complementary DNA) and the like are integrated on a glass substrate or a silicon substrate, exhaustive analyses of hybridization become possible.

With the DNA chip, the usual practice is that a target nucleic acid, labeled with a fluorescent dye beforehand, is subjected to hybridization with a probe nucleic acid fixed on a substrate, and the result of the interaction is taken and detected as a fluorescence amount (fluorescent signal).

In this fluorescent detection technique, a background noise that generates from a single-stranded probe nucleic acid not hybridized with a target nucleic acid gives an influence to such an extent as not negligible for detection accuracy. More particularly, how far a so-called S/N ratio is improved by excluding noise fluorescence, which is substantially irrelevant to hybridization, from a fluorescent signal to be detected directly leads to an improvement in accuracy of hybridization detection.

To this end, it is now general to adopt the step of removing by rinsing the single-stranded nucleic acid, which is present on the substrate and causes the generation of noise fluorescence, from the substrate. In Japanese Patent Laid-open No. 2003-84002 (see, especially, claim 1), there is proposed a technique using a quenching agent so as to reduce a background noise in the course of fluorescent detection by use of a DNA chip (DNA microarray). In JP-A-2004-519689T (see, especially, claim 1), there is proposed a method wherein a probe nucleic acid, not hybridized, is removed from a substrate by digestion with a nuclease.

Recently, "intercalator" which specifically binds to an interspace between complementary strands of a two-stranded nucleic acid to emit fluorescence has been developed, and a hybridization detection technique using this intercalator has been proposed. This detection technique has an advantage in that no preparation of a fluorescence-labeled target nucleic acid is necessary and thus, is considered to widely prevail in the future.

It is known that an intercalator tends to nonspecifically bind to a single-stranded nucleic acid that is free in a reaction region, a surplus single strand moiety of a counter nucleic acid forming a complementary strand, or a loop-forming moiety thereby emitting fluorescence although a significant difference is involved in the binding amount (e.g. H.S. Rye, A.N. Glazer, Nucleic Acid Research, 1995, Vol. 23, No. 7, 1215-1222).

In the hybridization detection using an intercalator, to reduce background noise fluorescence also becomes an important technical problem. If there is made an attempt to solve this problem by a procedure of removing, from a substrate by rinsing, a free single-stranded target nucleic acid or the like not taking part in the hybridization, like the conventional detection technique using a fluorescence-labeled target nucleic acid, an additional assay step is needed, thus taking extra labor and the design for rinsing conditions has to be made exactly. Moreover, there may be assumed an adverse influence such as of dissociation of a normally hybridized duplex upon exposure to rinsing.

Accordingly, the invention has for its primary object the provision of a novel and useful technique capable of achieving an improved S/N ratio in a hybridization detection technique using an intercalator.

### [Disclosure of the Invention]

The invention provides a method for detecting a hybridization by measuring a fluorescence intensity from an intercalator binding to complementary strand sites of a probe nucleic acid and a target nucleic acid, the hybridization detection method including, at least, the step of digesting, with an enzyme having nuclease activity, a single-stranded nucleic acid or/and a nucleic acid moiety inducing generation of noise fluorescence due to the nonspecific binding of the intercalator.

The "single-stranded nucleic acid" that is an objective to be digested with the enzyme is, for example, a single-stranded target nucleic acid not taking part in the hybridization, and this single-stranded target nucleic acid includes one that is free in a medium of a reaction region and one that is in a state of nonspecific adsorption on a solid phase surface such as of a substrate or bead. Besides, for "single-stranded nucleic acid" that is an objective to be digested with the enzyme, mention can be made of a probe nucleic acid which is present as a single-stranded state in the reaction region without taking part in the hybridization. It will be noted that the probe nucleic acid includes both of an acid that is used after fixing on a solid phase surface such as of a substrate or bead at terminal ends thereof and an acid used in a free state in a reaction region, and should not be construed as limited narrowly.

Next, as the "nucleic acid moiety", which is an objective to be digested with an enzyme, mention can be made, for example, of a surplus single-stranded nucleic acid moiety of a target nucleic acid, which has a strand longer than a probe nucleic acid, in the target nucleic acid constituting the double stranded nucleic acid formed by hybridization, and a single stranded nucleic acid moiety forming a self-loop structure. Either or both of these nucleic acid moieties are simultaneously digested and degraded with an enzyme having the nuclease activity.

The "enzyme" usable in the present invention is an enzyme having the digestion and degradation action of a nucleotide chain, i.e. an enzyme having the nuclease activity. Examples include an exonuclease selectively acting on a singe-stranded nucleic acid for degradation in a direction of 3'→5', an exonuclease selectively acting on a single-stranded nucleic acid for degradation in a direction of 5'→3', and an exonuclease selectively acting on a single-stranded nucleic acid for bidirectional degradation in directions of 3'→5' and 5'→3'. Alternatively, an endonuclease undergoing digestion and degradation from an inside site of a nucleic acid molecule maybe adaptable depending on the purpose and, in some case, an exonuclease and an endonuclease may be used in combination.

The exonuclease is suited for digestion and degradation of a target nucleic acid that does not take part in hybridization and is present in a single-stranded state, or a surplus singe-stranded nucleic acid moiety in a target nucleic acid making up of the double-stranded nucleic acid formed by hybridization. Choice of either an exonuclease that degrades the acid in a direction of 3'→5' or an exonuclease that degrades in a direction of 5' →3' can be determined on whether the free terminal end of a single-stranded nuleic acid, which is an objective of digestion and degradation, is a 3' terminal end or a 5' terminal end. In case where both 3' terminal end and 5' terminal end are present in the reaction region as a free terminal end of a single-stranded nucleic acid, an exonuclease degrading in a direction of 3'→5' and an exonuclease degrading in a direction of 5'→3' are used in combination, or an exonuclease bidirectionally degrading in directions of 3'→5' and 5'→3' is adopted.

For instance, assuming that a fixed probe nucleic acid present as a single-stranded state in the reaction region is digested and degraded, an exonuclease degrading in a direction of 3'→5' is chosen for the case of being fixed at the 5' terminal side and, in contrast, an exonuclease degrading in a direction of 5'→3' is chosen for the case of being fixed at the 3' terminal side.

If there are mixed, in a reaction region, a single-stranded nucleic acid having a free 3' terminal end, a single-stranded nucleic acid moiety having the same terminal end as indicated above, a single-stranded nucleic acid having a free 5' terminal end, and a single-stranded nucleic acid moiety having the same terminal end, both an exonuclease degrading toward a direction of 3'→5' and an exonuclease degrading toward a direction of 5'→3' are used in combination, or an exonuclease having the action of bidirectional degradation in directions of 3'→5' and 5'→3' is used.

The main technical terms related to the invention are illustrated.

"Probe nucleic acid" means a nucleic acid molecule that is present in a medium stored or kept in a reaction region and functions as a probe (detector) for detecting a target molecule specifically interacting with the acid molecule and may be present in a free state in the reaction region that is a field of hybridization or may be present as fixed to the solid phase surface such as of a substrate or bead, at one end thereon. For instance, typical examples include an oligonucleotide or polynucleotide such as a DNA probe.

"Target nucleic acid" means a nucleic acid molecule prepared as a sample for the purpose of checking whether a complementary base pair is formed between with the probe nucleic acid. It will be noted that the nucleotide chain length of this target nucleic acid is generally longer than a nucleotide chain length of a probe nucleic acid, for which if a complementary strand is formed between with the probe nucleic acid, a surplus single-stranded nucleic acid moiety occurs at the side of the target nucleic acid.

"Nucleic acid" means a polymer (nucleotide chain) of a phosphoric acid ester of a nucleoside formed through glucoside bonding between purine or a pyrimidine base and sugar and widely includes an oligonucleotide including a probe DNA, a polynucleotide, a DNA of a purine nucleotide and a pyrimidine nucleotide being polymerized (its full length or a fraction thereof), a cDNA (c probe DNA) obtained by reverse transcription, RNA and a polyamide nucleotide analog (PNA) and the like.

"Hybridization" means a complementary strand (double strand) formation reaction during the course of provision of a complementary base sequence structure.

"Nuclease activity" means an enzymatic activity of digesting and degrading a nucleic acid. "Exonuclease" is a kind of nuclease (nuclease) and is an enzyme which produces a mononucleotide by successively cutting 3',5'-phosphodiester bonds from one end of a nucleotide chain such as a DNA chain or an RNA chain. "Endonuclease (endonuclease)" is an enzyme producing an oligopolynucleotide by cutting 3',5'-phosphodiester bonds inside a nucleotide chain such as a DNA chain or an RNA chain.

"Self-loop structure" means a structural portion that projects to form a loop shape in part of a single-stranded or double-stranded nucleotide chain and includes both types with and without a complementary base pair moiety.

"Noise fluorescence" means fluorescence which becomes a background noise in the fluorescence detection of hybridization. In the practice of the invention, relative to normal fluorescence emitted from an intercalator specifically bound to a complementary strand obtained as a result of hybridization, fluorescence emitted from an intercalator nonspecifically bound to a nucleic acid site other than the complementary strand is called noise fluorescence.

"Intercalator" is a substance which emits fluorescence through combination with a complementary strand of a double-stranded nucleic acid and is used for hybridization detection. For instance, mention is made of POPO-1, TOTO-3, SYBR (registered trademark) Green I and the like.

According to the invention, in the method of detecting hybridization by measuring a fluorescence intensity from an intercalator binding to complementary strand sites of a probe nucleic acid and a target nucleic acid, the noise fluorescence can be caused to disappear, so that the hybridization can be quantitatively detected based on good S/N. After permitting an interaction such as hybridization to proceed, a rinsing operation with an aqueous solution for removing a substance causing the noise fluorescence to occur can be omitted.

### [Brief Description of the Drawings]

[Fig. 1] Fig. 1 is a view illustrating a fundamental principle and framework of a hybridization detection method according to the invention.
[Fig. 2] Fig. 2 is a view schematically showing an example of a typical hybridization assay state in a reaction region (R) in the vicinity of a solid phase surface (S) such as of a substrate.
[Fig. 3] Fig. 3 is a view schematically showing another hybridization assay state in the same reaction region (R).
[Fig. 4] Fig. 4 is a graph and a data sheet substituted for a drawing and showing the results of Example 1.

### [Best Mode for Carrying out the Invention]

Preferred embodiments of the invention are now described with reference to the accompanying drawings. It will be noted that the respective embodiments illustrated in the attached drawings exemplify typical embodiments and should not be construed as narrowly limiting the scope of the invention thereto.

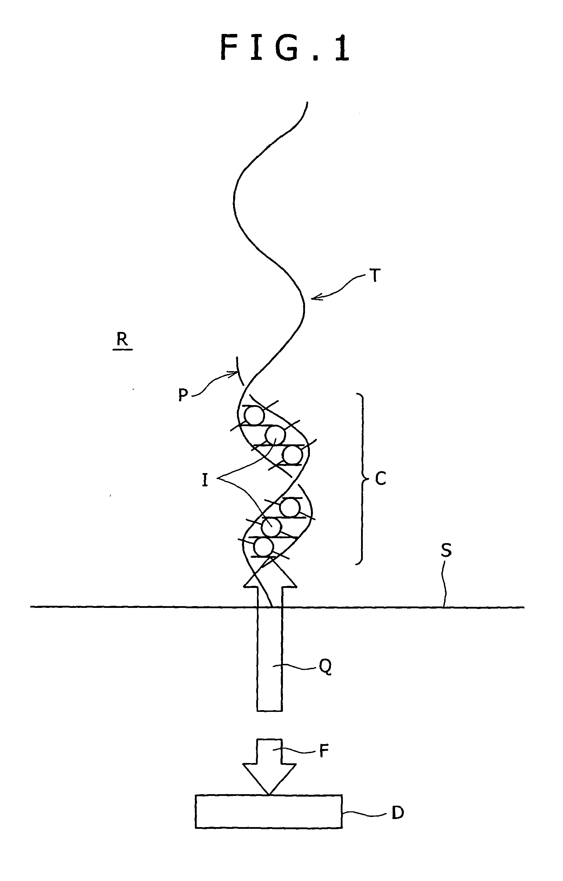

Fig. 1 is a schematic view showing a fundamental principle and framework of a hybridization detection method according to the invention.

In Fig. 1, there is shown a state of forming a complementary strand C as a result of the progress of hybridization between probe nucleic acid P, which is fixed at one terminal end thereof (3' end is exemplified in Fig 1) against a solid phase surface (e.g. a substrate or bead) indicated by symbol S, and target nucleic acid T added to a reaction region R wherein the probe nucleic acid is present.

It will be noted that in the practice of the invention, the terminal end is not critically limited to the 3' terminal end and may be a 5' terminal end (not shown). The probe nucleic acid P is not limited to the use in a fixed state, and the invention may also be applicable to an assay that is carried out in such a way that probe nucleic acid P is in a free state in the reaction region R. A medium stored or kept in the reaction region R is not critical and a wide variety of media may be adopted including, for example, solvents, gels, mixtures of solvents and gels.

Next, symbol I in Fig. 1 indicates an intercalator emitting fluorescence by specifically binding to the complementary strand C portion obtained by the hybridization. In the hybridization detection method according to the invention, fluorescent excitation light Q is focused and irradiated, in the reaction region R, from an outer light source (e.g. a laser beam source with a given wavelength) not shown and an intensity of excited fluorescence F (fluorescence amount) is measured according to an optical detection means D such as a known photodetector.

The intercalator I can nonspecifically bind to a single-stranded nucleic acid freed in the reaction region R, a surplus single-stranded moiety of a nucleic acid forming one side of the complementary strand (e.g. a target nucleic acid whose strand is longer than the probe nucleic acid P), and a nucleic acid moiety forming a self-loop structure, and thus, fluorescence (i.e. noise fluorescence) is emitted by the bindings.

In accordance with the invention, while not reducing a normal fluorescence amount under conditions of keeping the binding of the intercalator to the complementary strand, nucleotide chains serving as a generation source of noise fluorescence are digested and degraded by making effective use of the cutting action of nucleic acids with an enzyme having the nuclease activity such as a nucleolytic enzyme. In this way, it is an important purpose to reduce or extinguish the noise fluorescence from the reaction region R. This is particularly illustrated by giving examples.

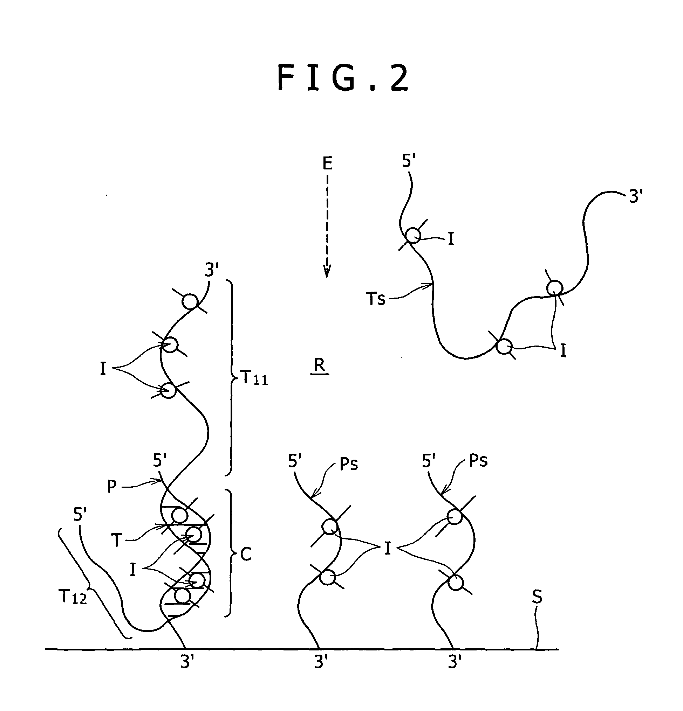

Fig. 2 is a view schematically illustrating an example of a typical hybridization assay state in a reaction region R provided in the vicinity of a solid phase surface S such as a substrate.

A probe nucleic acid P is fixed at a 3' terminal end of a solid phase surface S, to which a target nucleic acid T is hybridized to form a complementary stand C (see Fig. 2). Since the nucleotide chain length of the target nucleic acid T is longer than the nucleotide chain length of the probe nucleic acid P (see Fig. 2), for which there occur a single-stranded nucleic acid moiety T₁₁ with a surplus chain length at the 3' terminal end side of the target nucleic acid T and also a single-stranded nucleic acid moiety T₁₂ with a surplus chain length at the 5' terminal end side.

In Fig. 2, there are also shown a single-stranded target nucleic acid Ts which is present in a free state in the reaction region R and does not take part in (or cannot take part in) the hybridization and also a single-stranded probe nucleic acid Ps.

The reason why the single-stranded target nucleic acid Ts and the single-stranded probe nucleic acid Ps do not take part in the hybridization is considered for the case where there is no base sequence moiety forming a complementary strand in the nucleotide chain and also for the case where even if such a complementary base sequence moiety is present, no hybridization can take place by the influence of steric hindrance.

If the intercalator I is added to the reaction region R in such a state as set out above, the intercalator I binds specifically to the complementary strand C portion and also nonspecifically to the single-stranded nucleic acid moieties T₁₁, T₁₂, free single-stranded target nucleic acid Ts and single-stranded probe nucleic acid Ps although the binding amounts differ from each other.

In such a case as set out above, although the fluorescence induced from the intercalator I binding to the complementary strand C moiety provides a normal fluorescence signal useful for the hybridization detection, the fluorescence induced from the intercalator I nonspecifically binding to the single-stranded nucleic moieties T₁₁, T₁₂, free single-stranded target nucleic acid Ts and single-stranded probe nucleic acid Ps becomes a background noise, thereby causing the S/N ratio to be worsened.

In this embodiment, when enzyme E such as an exonuclease having the selective digestion and degradation action of a single-stranded nucleic acid in a direction of 3'→5' is added to the reaction region R, the single-stranded nucleic acid moiety T₁₁ or free single-stranded target nucleic acid Ts of the target nucleic acid T is digested and degraded (cut) from the 3' terminal end side to remove the binding of the intercalator thereby causing the noise fluorescence to disappear.

Further, when enzyme E such as an exonuclease having the selective digestion and degradation action of the single-stranded nucleic acid in a direction of 5'→3' is added to the reaction region R, the single-stranded nucleic acid moiety T₁₂ or free single-stranded target nucleic acid Ps of the target nucleic acid T is digested and degraded (cut) from the 5' terminal end side to remove the binding of the intercalator thereby causing the noise fluorescence to disappear.

Alternatively, if enzyme E such as an exonuclease having the bidirectional degradation action in directions of 3'→5' and 5'→3' is added to the reaction region R, all the single-stranded nucleic acid moieties T₁₁, T₁₂, free single-stranded target nucleic acid Ts and single-stranded probe nucleic acid Ps are digested and degraded from both directions of the 3' terminal end side and the 5' terminal end side thereby causing the noise fluorescence to disappear.

According to these methods, the fluorescence amount induced from the intercalator I binding to the complementary stand C portion, that is, a ratio of noise fluorescence to the fluorescence amount useful for the hybridization detection or an S/N ratio, can be improved.

It will be noted that in the state of the reaction region R shown in Fig. 2, although the probe nucleic acid X fixed to the solid phase surface at the 3' terminal end is exemplified, this is true of the case of a probe nucleic acid X fixed to the solid phase surface at the 5' terminal end (not shown).

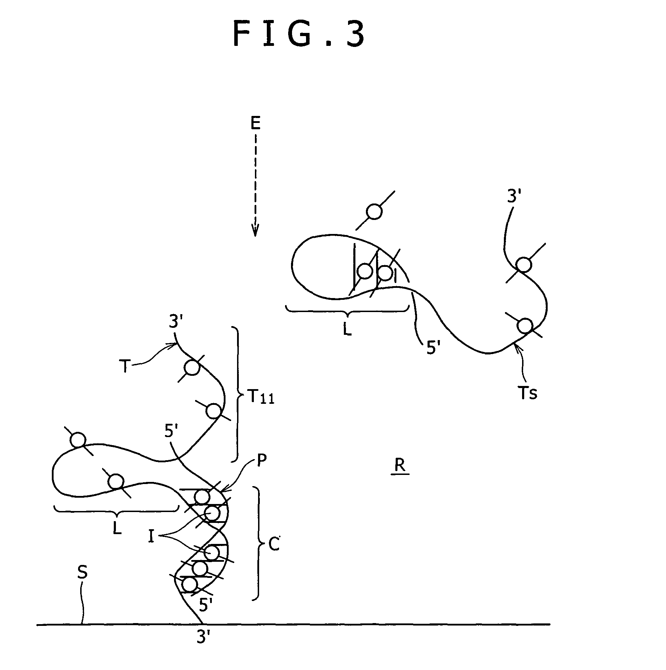

Next, Fig. 3 is a view schematically showing another hybridization assay state in the reaction region R.

In Fig. 3, there is shown, along with a probe nucleic acid P fixed to a solid phase surface S, a target nucleic acid T which forms a complementary strand C together with the probe nucleic acid P and also forms a self-loop structure L at part thereof.

In addition, in Fig. 3, there is shown a target nucleic acid Ts forming a self-loop structure at part thereof, which is free in a reaction region R with no involvement in hybridization.

In order to reduce or erase noise fluorescence in such a state of the reaction region R as set out above, a single-stranded nucleic acid moiety T₁₁ of the target nucleic acid T forming the complementary strand C along with the probe nucleic acid P, the loop structure L portion of the target nucleic acid T and a single target nucleic acid Ts that is present in a free state and has a loop structure L at part thereof are digested and degraded with an enzyme having the nuclease activity. For instance, an exonuclease and an endonuclease are added to the reaction region R.

The enzyme having the nuclease activity usable in the invention includes, for example, an exonuclease (Exonuclease), an endonuclease (Endonuclease) or a polymerase (Polymerase) or a mixture thereof.

For "exonuclease", mention can be made, for example, of exonuclease I, exonuclease VII, nuclease Bal 31 and the like. Exonuclease I selectively acts on a single-stranded DNA for degradation in a direction of 3'→5' thereby forming a 5'-monomer. Exonuclease VII specifically acts on a single-stranded DNA and is suited for elimination of a single-stranded terminal portion of a double-stranded DNA. Nuclease Bal 31 simultaneously digests and degrades both strands at the 3' and 5' terminal ends of a double-stranded DNA thereby producing a shorter double-stranded DNA, and has the action as an endonuclease for a single-stranded nucleic acid.

For "endonuclease", mention can be made, for example, of nuclease S₁, nuclease P₁, mung bean nuclease (nuclease of mung bean) and the like. For instance, klenow enzyme (Klenow Fragment) having the nuclease activity and the like can be mentioned for a method of utilizing a polymerase.

It will be noted that the nuclease S₁ acts on a single-stranded nucleic acid and can be utilized to remove a single-stranded portion in a double-stranded nucleic acid or DNA-RNA hybrid nucleic acid or to cut a portion which becomes a single-stranded loop structure without a complementary portion and runs over outside. The nuclease P₁ is a Zn²⁺-dependent endonuclease which is able to degrade a single-stranded DNA or RNA into a 5'-nucleotide and is useful for selective degradation of a single-stranded nucleic acid like nuclease S₁.

Where the probe nucleic acid is RNA and the target nucleic acid T is DNA, or where the probe nucleic acid is DNA and the target nucleic acid is RNA, or where both probe nucleic acid and target nucleic acid are made of RNA, a ribonuclease acting on RNA (RNase), a deoxyribonuclease (DNase) acting on DNA, a mixture thereof, or a nuclease acting on both DNA and RNA may be, for example, used depending on the purpose or necessity. In some cases, ribonuclease H activity degrading a RNA strand of DNA-RNA hybrid chain may be used.

The manner of selecting an enzyme, the quantity of an enzyme, the time for enzymatic reaction, the temperature for the enzymatic reaction and the like conditions as set out hereinabove should be so selected as to enable the nucleotide chain to be digested and degraded (cut) at least to a length of a nucleic acid incapable of binding of the intercalator thereto. The enzyme should be selected to satisfy the conditions of (1) no adsorption of the intercalator on the enzyme per se, (2) no lowering of the enzymatic activity in coexistence of the intercalator, and (3) no inhibition of binding of the intercalator to the complementary chain C.

The buffer conditions in the step of carrying out digestion and degradation of a nucleotide chain with enzymes are not required to be recommending conditions for individual enzymes. For example, when the intercalator is SYBR Green I and an enzyme used is nuclease T4 or klenow fragment (Klenow Fragment), it is possible to use an HEPES buffer solution with a pH of 7.0 - 8.5 to which MgCl₂ is added.

Where the nucleotide chain to be digested and degraded with an enzyme is long and has a self-loop structure, it is possible to charge an enzyme for the digestion and degradation and a protein specifically binding to a single strand at the same time.

The charge timing of an enzyme into the reaction region R is not critical and may be at any of stages prior to addition of the intercalator, simultaneous with addition of the intercalator, and after addition of the intercalator. If there is a concern about the influence of the intercalator bound to a nucleic acid on the action of the enzyme, it is desirable to carry out the digestion and degradation such as of a single-stranded nucleic acid and the like with an enzyme prior to addition of the intercalator.

In the practice of the invention, the probe nucleic acid Ps alone in a single-stranded state, as shown in Fig. 2, can be excluded from an objective for enzymatic treatment by proper selection of an enzyme, followed by addition of the intercalator I to the reaction region R, thereby checking a noise fluorescence amount induced from the probe nucleic acid Ps.

More specifically, assuming the case where no surplus single-stranded nucleic acid moiety T₁₂ is present at the 5' terminal end side of the target nucleic acid T in the state shown in Fig. 2, addition of an exonuclease in a direction of 3'→5' or a double-stranded specific nuclease enables nucleotide chains present in the reaction region R to be limited only to the probe nucleic acid Ps. In this condition, if an intercalator is added, the resulting fluorescence can be obtained as a noise fluorescence amount induced from the probe nucleic acid Ps. Accurate determination of such a probe nucleic acid Ps-induced noise fluorescence amount is effective for use as basic data useful for measurement of hybridization.

### [Example 1]

A check test concerning nonspecific binding of an intercalator (SYBR Green I) to a single-stranded DNA in a liquid phase was conducted.

Experimental method: one type of oligonucleotide was added to a 0.1 M phosphate buffer (NaCl 200 ml) at a pH of 7.4 at a concentration of 10 nM. The resulting sample solution was placed in an incubator of 10°C so that the temperature of the sample solution was set at 10°C. Next, SYBR Green I was added to the sample solution to a 1:10000 dilution, and the sample solution was allowed to stand in the incubator of 10°C, followed by fluorescence measurement (F-4500, made by Hitachi Ltd., with an excitation wavelength of 497 nm).

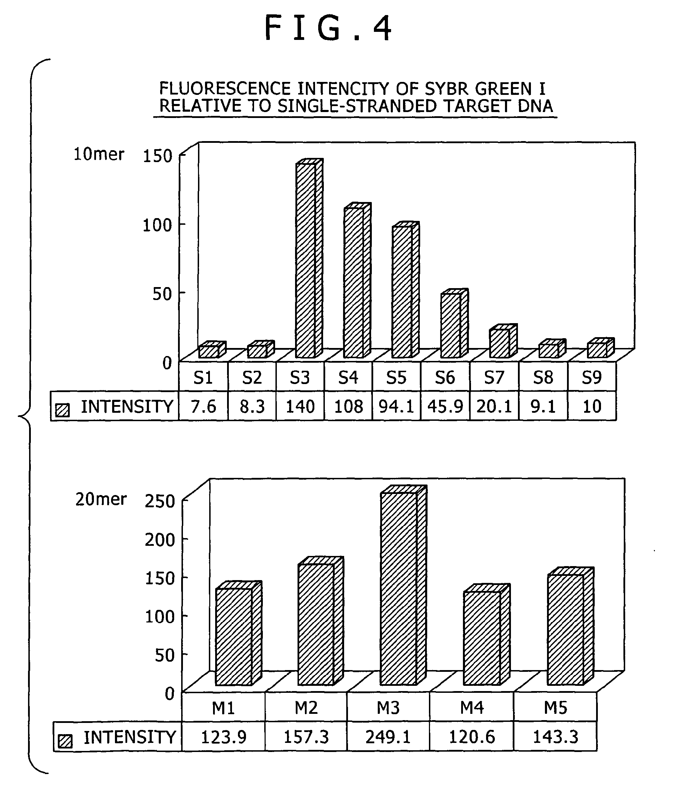

The setting conditions concerning the probe DNA and target DNA used in this test are as shown in "Table 1" below, and the results of the experiment are shown in Fig. 4.

**[Table 1]**

| [Oligonucleotides used in the experiment] | | |
|---|---|---|
| Length | Name | Sequence |
| 10 mer | S1 | 5'-d(CTCCCCTTTC) |
| | S2 | 5'-d(GAAAGGGGAG) |
| | S3 | 5'-d(CCCTGGGGGC) |
| | S4 | 5'-d(CCCAAAATGG) |
| | S5 | 5'-d(CCATTTTGGG) |
| | S6 | 5'-d(GCAGCGCCTC) |
| | S7 | 5'-d(GGTTCATGCC) |
| | S8 | 5'-d(GTGATGATGG) |
| | S9 | 5'-d(CCATCATCAC) |
| 20 mer | M1 | 5'-d(GGCAGCGCCTCACAACCTCC) |
| | M2 | 5'-d(GGAGGTTGTGAGGCGCTGCC) |
| | M3 | 5'-d(GGAGGTTGCGAGGCGCTGCC) |
| | M4 | 5'-d(GGAGGTTGTGAGGCACTGCC) |
| | M5 | 5'-d(GGAGGTTGCGAGGGACTGCC) |

As will be apparent from the results shown in Fig. 4, judging from the intercalator fluorescence values of the 10-mer (S1-S9 in Table 1) and 20-mer (M1-M5 in Table 1) single-stranded DNA (oligonucleotide), SYBR Green I that is an intercalator can generate noise fluorescence by nonspecific binding to the single-stranded target DNA. It will be noted that the single-20-mer single-stranded DNA was higher than the 10-mer single-stranded DNA with respect to the fluorescence value.

Accordingly, it could be confirmed that it was necessary to inhibit the nonspecific binding of SYBR Green I to the single-stranded target DNA.

### [Example 2]

An experiment for checking a difference in fluorescence value was carried out wherein using target DNA fragments 1, 2 having different chain lengths, hybridization was caused to proceed with respect to a probe oligo DNA fixed to a solid phase surface (substrate).

Experimental method: Avidin (Avidin) was fixed to an Au electrode of a sensor chip for a crystal oscillator-based QCM device (AFFINIX Q, made by Initium Inc.) according to a recommending procedure for the device. Next, a probe DNA modified with biotin (biotin) at the 5' terminal end thereof was fixed on the sensor chip through the avidin-biotin binding. Subsequently, the sensor chip was rinsed to remove a free probe DNA present without fixing to the reaction region. Thereafter, a target DNA was added thereby causing hybridization to proceed, followed by rinsing again to remove a free target oligo DNA present without undergoing hybridization in the reaction region. After the removing step, SYBR Green I serving as an intercalator was added and intercalated with the double strand.

Setting conditions on the concentrations of the probe DNA, target DNA and oligo DNA and the dilution rate of the intercalator are as shown in the following "Table 2". The results of this experiment are as shown in the following "Table 3".

**[Table 2]**

| Experimental conditions | |
|---|---|
| Probe DNA (20 mer) | 5'-d(CGAAGCGCTTATTCCAGAGC) |
| Target DNA 1 (20 mer) | 5'-d(GCTCTGGAATAAGCGCTTCG) |
| Target DNA 2 (40 mer) | 5'-d(CACGTATCCATTCATGTCGGGCTCTGGAATAAGCGCTTCG) |
| Dilution rate of SYBR Green I | 1:10000 dilution |

**[Table 3]**

| [Experimental results] | | |
|---|---|---|
| Oligo DNA used as an objective for fluorescence measurement | Fixed amount (pmols) of probe DNA | Binding amount of SYBR Green I (Hz) |
| Probe DNA/target DNA 1 (20 mer/20 mer) | 0.74 | 20.5 |
| Probe DNA/target DNA 2 (20 mer/40 mer) | 0.81 | 51.2 |

From the results indicated in the above "Table 3", it has been confirmed that when the chain length is such that target DNA > probe DNA, SYBR Green I binds to the surplus single-stranded moiety of the target DNA to emit fluorescence serving as a background. Accordingly, in order to increase the accuracy of the hybridization detection, it has been found that elimination of the binding of SYBR Green I to the surplus single-branded moiety of the target DNA forming the complementary strand is needed.

### [Example 3]

An experiment was carried out so as to check a variation in fluorescence amount depending on the digestion and degradation of a single-stranded DNA with exonuclease VII.

Experimental method: a probe DNA and a target DNA 1 or target DNA 2 was added to a 0.1 M phosphate buffer (NaCl 200 mM) with a pH of 7.4 so that a concentration thereof was at 100 nM. Besides, a system in which the probe DNA alone was added so that its concentration was at 100 nM was also provided. Next, after annealing (cooling 5 minutes after conditions of 95°C), exonuclease VI was added, followed by incubation at 37°C for one hour to cause an enzymatic reaction to proceed. Thereafter, SYBR Green I was added to the resulting sample solution at 1:10000 dilution and the sample solution was allowed to stand in an incubator of 10°C, followed by fluorescence measurement (F-4500, made by Hitachi ltd., with an excitation wavelength of 497 nm).

Setting conditions on the concentrations of the probe DNA, target DNA and oligo DNA and the dilution rate of the intercalator are as shown in the following "Table 4". The results of this experiment are as shown in the following "Table 5".

**[Table 4]**

| Experimental conditions | |
|---|---|
| Probe DNA | 5'-d(CGAAGCGCTTATTCCAGAGC) |
| Target DNA 1 Target DNA 2 | 5'-d(GCTCTGGAATAAGCGCTTCG) |
| | 5'-d(CACGTATCCATTCATGTCGGGCTCTGGAATAAGCGCTTCG) |
| Concentration of oligo DNA | 100 mM |
| Dilution rate of SYBR Green I | 1:10000 dilution |

**[Table 5]**

| [Experimental results] | | |
|---|---|---|
| Oligo DNA used an objective for fluorescence measurement | Fluorescence value (prior to enzymatic digestion) | Fluorescence value (after enzymatic digestion) |
| Probe DNA/target DNA 1 (20 mer/20 mer) | 702 | 799±67 |
| Probe DNA/target DNA 2 (20 mer/40 mer) | 551.2 | 770±38 |
| Target DNA | 74 | 4.3±1.7 |

From the results indicated in the above "Table 5", the fluorescence values are almost same as each other prior to and after the enzymatic treatment with respect to the double-stranded DNA of a complete complementary strand. This indicates that the exonuclease VII specifically acts on the single-stranded DNA and has no digestion and degradation action on the double-stranded DNA that is a complete complementary strand.

On the other hand, with probe DNA/target DNA 2 (20 mer/40 mer), the fluorescence value after completion of the enzymatic digestion became the same as with the probe DNA/target DNA 1 (20 mer/20 mer) (see Table 5). This is considered for the reason that according to the enzymatic treatment, the surplus single-stranded target DNA moiety of the target DNA 2 is cut and the SYBR Green I released by the treatment binds to the complementary strand to increase the fluorescence amount.

When the single-stranded target DNA was treated with exonuclease VII, the resulting fluorescent value became nearly zero (see "Table 5"). This is considered as follows: the single-stranded target DNA is digested and degraded with the oligo DNA whose chain length is shorter, for which SYBR Green I does not nonspecifically bind thereto.

It will be noted that in this experiment, the fluorescence value was measured by addition of SYBR Green I after the enzymatic treatment. More particularly, fluorescence measurement is conducted in the mixture of degradation product of oligo DNA, an enzyme, and SYBR Green I after completion of the enzymatic treatment. This gives evidence that even if a digestion and degradation product of oligo DNA, an enzyme, and SYBR Green I are mixed, appropriate fluorescence measurement can be made.

According to a supplementary experiment, it has been confirmed that where SYBR Green I is mixed upon treatment with exonuclease VII, there is no inhibition of the digestion and degradation action with this enzyme. Hence, SYBR Green I does not serve as a inhibitor for enzymatic activity, for which it has been found possible that simultaneous addition with exonuclease VII leads to a reduced number of steps.

### [Industrial Applicability]

The invention can be utilized for a technique of detecting hybridization by measuring fluorescence intensity from an intercalator binding to complementary strand moieties of a probe nucleic acid and a target nucleic acid. Especially, this technique can be used to reduce or extinguish noise fluorescence, thereby improving S/N. The invention can also be utilized as a hybridization detection technique without carrying out a step of rinsing, with an aqueous solution, noise fluorescence causal molecules, i.e. a rinsing-free hybridization detection technique.

## Claims

1. A hybridization detection method by measuring a fluorescence intensity from an intercalator binding to complementary strand sites of a probe nucleic acid and a target nucleic acid, comprising,
at least, the step of digesting, with an enzyme having nuclease activity, a single-stranded nucleic acid or/and a nucleic acid moiety inducing generation of noise fluorescence due to nonspecific binding of the intercalator.

2. The hybridization detection method according to Claim 1, wherein said single-stranded nucleic acid is either or both of (1) and (2) indicated below
(1) a single-stranded target nucleic acid that does not take part in the hybridization, and
(2) a single-stranded probe nucleic acid that does not take part in the hybridization.

3. The hybridization detection method according to Claim 1, wherein said nucleic acid moiety is either or both of (a) and (b) indicated below
(a) a surplus single-stranded nucleic acid moiety of a target nucleic acid constituting a double-stranded nucleic acid formed by the hybridization, and
(b) a single-stranded nucleic acid moiety forming a self-loop structure.

4. The hybridization detection method according to Claim 1, wherein said enzyme is made of an exonuclease.

5. The hybridization detection method according to Claim 4, wherein said exonuclease selectively acts on a single-stranded nucleic acid for degradation in a direction of 3'→5'.

6. The hybridization detection method according to Claim 4, wherein said exonuclease selectively acts on a single-stranded nucleic acid for degradation in a direction of 5'→3'.

7. The hybridization detection method according to Claim 4, wherein said exonuclease selectively acts on a single-stranded nucleic acid for bidirectional degradation in directions of 3'→5' and 5'→3'.

8. The hybridization detection method according to Claim 1, wherein said nucleic acid degrading enzyme is made of endonuclease.

9. The hybridization detection method according to Claim 1, wherein said probe nucleic acid is fixed to a surface for detection of the hybridization.
